# EUROPÄISCHE PATENTANMELDUNG

(11) **EP 3 578 139 A1**
(43) Veröffentlichungstag der Anmeldung: **11.12.2019**
(21) Anmeldenummer: 18177063.7
(22) Anmeldetag: 11.06.2018
(51) Int. Cl.: A61F 2/46

(54) **MODULARES CHIRURGISCHES INSTRUMENT**

(30) Priorität: 06.06.2018 DE 202018103170 U
(71) Anmelder: Müller, Erich Johann, 63839 Kleinwallstadt (DE)
(72) Erfinder: Müller, Erich Johann, 63839 Kleinwallstadt (DE)
(74) Vertreter: Becker Kurig Straus

(57) **Zusammenfassung**

Bereitgestellt wird ein modulares chirurgisches Instrument zum Ausschlagen oder Einsetzen einer Prothese, die eine Prothesenachse definiert und die einen Prothesenhals aufweist, der eine Prothesenhalsachse definiert, die nicht parallel zur Prothesenachse verläuft, umfassend einen Werkzeugkopf mit einem Durchgang zur Aufnahme des Prothesenhalses, einer Verriegelungseinrichtung zur Arretierung des aufgenommenen Prothesenhalses und einer Spannaufnahme, die eine Achse aufweist, zum Übertragen einer Spannkraft für die Verriegelungseinrichtung. Der Durchgang und die Spannaufnahme sind in Bezug auf eine durch die Achse der Spannaufnahme definierten Längsrichtung an entgegengesetzten Enden des Werkzeugkopfes angeordnet, wobei der Durchgang eine Durchgangsachse definiert, die bei arretiertem Prothesenhals im Wesentlichen mit der Prothesenhalsachse zusammenfällt. Die Achse der Spannaufnahme ist von der durch die Längsrichtung und die Durchgangsachse definierten Ebene beabstandet und/oder ist von einer Durchgangs-Längsachse, die in Längsrichtung und durch den Durchgang verläuft, beabstandet. Weiterhin wird bereitgestellt ein Schlagrahmen für ein modulares chirurgisches Instrument zum Ausschlagen oder Einsetzen einer Prothese, die eine Prothesenachse definiert und die einen Prothesenhals aufweist, der eine Prothesenhalsachse definiert, die nicht parallel zur Prothesenachse verläuft, aufweisend einen ersten und einen zweiten Schenkel; wobei an einem Ende des ersten Schenkels eine erste Verbindungseinrichtung mit einer ersten Achse für eine Schlagvorrichtung angeordnet ist, wobei an einem Ende des zweiten Schenkels eine zweite Verbindungseinrichtung mit einer zur ersten Achse parallelen zweiten Achse für eine Spannbaugruppe angeordnet ist, wobei ein Raum zwischen den Enden der Schenkel in Richtung der ersten und zweiten Achse freibleibt und wobei die erste Achse und die zweite Achse voneinander beabstandet sind.

## Beschreibung

### Gebiet der Erfindung

Die vorliegende Erfindung betrifft ein modulares universelles chirurgisches Instrument zum Ausschlagen oder Einsetzen von künstlichen Prothesen bzw. Gelenkprothesen, insbesondere Hüftprothesen, das bei unterschiedlichen minimal-chirurgischen Zugangsarten Verwendung findet.

### Stand der Technik

Seit mehreren Jahrzehnten werden künstliche Gelenkprothesen chirurgisch eingesetzt, z.B. als Hüft-, Knie-, oder Schultergelenkersatz. Allein in Deutschland sind etwa 400000 Hüft- und Knieprothesen eingesetzt. Die Lebensdauer künstlicher Prothesen ist durch Infektionen, aseptische Lockerung und Brüche begrenzt. So beträgt die durchschnittliche Lebensdauer einer Prothese in etwa 10 bis 15 Jahre und eine chirurgische Revisions-Inzidenz beträgt in den ersten 10 Jahren nach dem Einsetzen einer Prothese etwa 10%. Dies macht eine Ersetzung von Prothesen notwendig, wobei eine Krafteinwirkung möglichst nur in einer Richtung entlang einer Prothesenachse erfolgen sollte, um eine Schädigung umliegender Knochen durch Querkräfte zu vermeiden.

Beim Einsetzen und Ersetzen künstlicher Prothesen wird eine minimalinvasive Eingriffsweise bevorzugt, dadurch werden sowohl Hautschnitte als auch eine weitergehende Schädigung von Weichgewebe vermieden bzw. reduziert. Beim Hüftgelenkersatz haben sich minimalinvasive Zugänge durchgesetzt, da lange Hautschnitte, das Ablösen von Muskeln und eine weitgehende Schädigung der Weichteile, die das Gelenk umgeben, vermieden werden. Z.B. werden die für das Gehen wichtigen Muskelgruppen an der seitlichen Hüfte bei minimalinvasiven Eingriffen geschont, so dass eine raschere und schmerzfreiere Mobilisierung, also Steh- und Gehfähigkeit, nach der Operation ermöglicht wird. Auch treten langanhaltende Reizungen der umgebenden Schleimbeutel ebenso wie Weichteilverkalkungen nach Hüftprothesen-Operationen mit minimalinvasivem Zugangsweg seltener auf.

Der Zugangsweg bei minimalinvasiven Eingriffen hat sich beispielsweise bei Hüftprothesen-Operationen von einem anfänglich hinteren über einen immer mehr seitlichen nach einem schließlich vorderen Zugang verlagert, so dass inzwischen diese 3 Hauptzugänge in etwa gleichen Anteil an allen minimal-invasiven Eingriffen besitzen. Dies macht eine Anpassung und Erneuerung der bei Operationen verwendeten Werkzeuge notwendig.

Bekannt sind chirurgische Werkzeuge, die eine Stellvorrichtung mit komplexem Aufbau aus vielen Einzelteilen und eine eng daran angeschlossene Bedienvorrichtung für die Festsetzung der Prothese aufweisen. Hierdurch erhält der Chirurg nur ein enges Arbeitsumfeld und Sichtfeld, wodurch das Einsetzen bzw. Entfernen der Prothese anfällig gegenüber Fehlern wird.

Zum Beispiel ist aus der DE 4332872 C1 ein Ausschlagwerkzeug mit einem Werkzeugkopf bekannt, der mittig zur Aufnahme eines Prothesenhalses geöffnet ist. Im Werkzeugkopf sind an der Aufnahme verschiebbare Stellglieder angeordnet, die durch eine anschließende Druckstange verschoben werden und so einen aufgenommenen Prothesenhals festklemmen können. In DE 29508578 U1 ist ein Werkzeugteil für ein solches Ausschlagwerkzeug offenbart, bei dem Stellglieder durch eine axial verschiebbare Druckstange eingestellt werden kann. Der Kopf des Werkzeugteils ist seitlich geschlossen.

Aufgabe der vorliegenden Erfindung ist also, ein modulares und universelles Ausschlaginstrument für unterschiedliche Zugangswege zu schaffen, mit unterschiedlichen Werkzeugköpfen, die für eine sichere Klemmung an unterschiedlichen Prothesenhälsen sorgen. Eine weitergehende Aufgabe besteht darin, zu gewährleisten, dass die Extraktionskraft lediglich in Prothesen-Axialrichtung wirkt und dass ein hinreichendes Arbeitsumfeld zur Verfügung steht, um ein Entfernen und Einsetzen von Prothesen ohne Prothesen-Knochenbettschädigung oder andere Fehler zu ermöglichen.

### Zusammenfassung der Erfindung

Die Aufgabe wird erfindungsgemäß gelöst durch ein modulares chirurgisches Instrument gemäß dem unabhängigen Anspruch 1. Weiter wird die Aufgabe erfindungsgemäß gelöst durch einen Schlagrahmen für ein modulares chirurgisches Instrument gemäß dem unabhängigen Anspruch 10. Abhängige Ansprüche definieren vorteilhafte Ausführungsformen.

Das modulare chirurgische Instrument zum Ausschlagen oder Einsetzen einer Prothese, die eine Prothesenachse definiert und die einen Prothesenhals aufweist, der eine Prothesenhalsachse definiert, die nicht parallel zur Prothesenachse verläuft, umfasst einen Werkzeugkopf mit einem Durchgang zur Aufnahme des Prothesenhalses, einer Verriegelungseinrichtung zur Arretierung des aufgenommenen Prothesenhalses und einer Spannaufnahme mit einer Achse zum Übertragen einer Spannkraft für die Verriegelungseinrichtung, wobei der Durchgang und die Spannaufnahme in Bezug auf eine durch die Achse der Spannaufnahme definierten Längsrichtung an entgegengesetzten Enden des Werkzeugkopfes angeordnet sind, und wobei der Durchgang eine Durchgangsachse definiert, die bei arretiertem Prothesenhals im Wesentlichen mit der Prothesenhalsachse zusammenfällt; wobei die Achse der Spannaufnahme von der durch die Längsrichtung und die Durchgangsachse definierten Ebene beabstandet ist und/oder die Achse der Spannaufnahme von einer Durchgangs-Längsachse, die in Längsrichtung und durch den Durchgang verläuft, beabstandet ist.

Gemäß einem Aspekt der vorliegenden Erfindung kann der Abstand der Achse der Spannaufnahme von der durch die Längsrichtung und die Durchgangsachse definierten Ebene im Bereich von 2 cm - 20 cm, bevorzugt 5 cm - 15 cm, liegen und/oder der Abstand der Achse der Spannaufnahme von der Durchgangs-Längsachse im Bereich von 2 cm - 20 cm, bevorzugt 5 cm - 15 cm, liegen.

Gemäß einem weiteren Aspekt kann das modulare chirurgische Instrument weiterhin eine Spannbaugruppe umfassen, die mit dem Werkzeugkopf an der Spannaufnahme lösbar verbunden ist und die ein Spannelement zur Beaufschlagung einer Spannkraft und Stellglieder zur Übertragung der Spannkraft aufweist.

Gemäß einem weiteren Aspekt kann das modulare chirurgische Instrument weiterhin einen Schlagrahmen umfassen, der mit der Spannbaugruppe lösbar verbunden ist und der eine erste Verbindungseinrichtung mit einer ersten Achse für eine Schlagvorrichtung zum Übertragen einer Schlagkraft aufweist.

Gemäß einem weiteren Aspekt kann das modulare chirurgische Instrument weiterhin die Schlagvorrichtung umfassen, die an der ersten Verbindungseinrichtung angebracht ist, wobei die Schlagvorrichtung eine Schlagplatte, insbesondere eine ebene Schlagplatte, oder einen Gleithammer umfasst und so eingerichtet ist, dass die Schlagkraft entlang der ersten Achse wirkt.

Gemäß einem weiteren Aspekt kann die Schlagvorrichtung eine konkave Schlagplatte umfassen.

Gemäß einem weiteren Aspekt kann die erste Verbindungseinrichtung oder eine feste Verbindung vom Durchgang aus gesehen in Längsrichtung angeordnet sein, so dass die erste Achse bei arretiertem Prothesenhals mit der Prothesenachse zusammenfällt.

Gemäß einem weiteren Aspekt kann der Schlagrahmen einen ersten und einen zweiten Schenkel umfassen, wobei an einem Ende des ersten Schenkels die erste Verbindungseinrichtung oder eine feste Verbindung, bevorzugt die Verbindungseinrichtung, mit der ersten Achse die Schlagvorrichtung angeordnet ist; wobei an einem Ende des zweiten Schenkels eine zweite Verbindungseinrichtung oder eine feste Verbindung, bevorzugt die Verbindungseinrichtung, mit einer zur ersten Achse parallelen zweiten Achse für die Spannbaugruppe angeordnet ist; und wobei ein Raum zwischen den Enden der Schenkel in Richtung der ersten und der zweiten Achse freibleibt.

Gemäß einem weiteren Aspekt können die erste Achse und die zweite Achse voneinander beabstandet sein.

Gemäß einem weiteren Aspekt kann der Schlagrahmen eine L-Form aufweisen, wobei die Spannbaugruppe an einem zweiten Schenkel der L-Form mittels einer zweiten Verbindungeinrichtung oder einer festen Verbindung lösbar verbunden ist und die erste Verbindungseinrichtung oder eine feste Verbindung für die Schlagvorrichtung an einem ersten Schenkel der L-Form angeordnet ist.

Gemäß einem weiteren Aspekt kann die Verriegelungseinrichtung ein in den Durchgang bewegbar eingreifendes Sperrglied zur Arretierung des Prothesenhalses umfassen, wobei die Durchgangsachse im Wesentlichen senkrecht zu einer Bewegungsrichtung des Sperrglieds verläuft.

Der Schlagrahmen für ein modulares chirurgisches Instrument zum Ausschlagen oder Einsetzen einer Prothese, die eine Prothesenachse definiert und die einen Prothesenhals aufweist, der eine Prothesenhalsachse definiert, die nicht parallel zur Prothesenachse verläuft, weist einen ersten und einen zweiten Schenkel auf; wobei an einem Ende des ersten Schenkels eine erste Verbindungseinrichtung mit einer ersten Achse für eine Schlagvorrichtung angeordnet ist; wobei an einem Ende des zweiten Schenkels eine zweite Verbindungseinrichtung mit einer zur ersten Achse parallelen zweiten Achse für eine Spannbaugruppe angeordnet ist; und wobei ein Raum zwischen den Enden der Schenkel (d.h. zwischen dem Ende des ersten Schenkels und dem Ende des zweiten Schenkels) in Richtung der Achsen freibleibt; wobei die erste Achse und die zweite Achse voneinander beabstandet sind.

Gemäß einem weiteren Aspekt des Schlagrahmens kann der Abstand zwischen der ersten Achse und der zweiten Achse im Bereich von 2 cm - 20 cm, bevorzugt 5 cm - 15 cm, liegen.

Gemäß einem weiteren Aspekt kann der Schlagrahmen mehrere Rahmenabschnitte umfassen, wobei der erste Schenkel und der zweite Schenkel Rahmenabschnitte bilden, deren Enden sich im Wesentlichen senkrecht zu der ersten und der zweiten Achse erstrecken, und wobei sich weitere Rahmenabschnitte, die den ersten Schenkel und den zweiten Schenkel verbinden, zumindest teilweise in die durch die erste und die zweite Achse definierte Richtung erstrecken.

Gemäß einem weiteren Aspekt des Schlagrahmens kann am ersten Schenkel zusätzlich eine dritte Verbindungseinrichtung, die bevorzugt ein Durchgangsloch am Schlagrahmen umfasst, mit einer dritten Achse für eine Schlagvorrichtung angeordnet sein, wobei die dritte Achse mit der zweiten Achse zusammenfällt.

Gemäß einem weiteren Aspekt des Schlagrahmens kann an der ersten Verbindungseinrichtung eine Schlagplatte oder ein Gleithammer angebracht sein.

Gemäß einem weiteren Aspekt des Schlagrahmens an der ersten Verbindungseinrichtung eine konkave Schlagplatte angebracht sein.

Gemäß einem weiteren Aspekt des Schlagrahmens können die erste und/oder die zweite Verbindungseinrichtung jeweils ein Durchgangsloch im Schlagrahmen umfassen.

Gemäß einem weiteren Aspekt des Schlagrahmens kann in dem Durchgangsloch bzw. den Durchgangslöchern jeweils eine Elastomerhülse, bevorzugt aus Silikon, angeordnet sein.

Gemäß einem weiteren Aspekt des Schlagrahmens kann die erste und/oder die zweite Verbindungseinrichtung eine Verbindungsschraube umfassen, die sich durch das Verbindungsloch erstreckt und die mittels einer Mutter befestigt wird.

Gemäß einem weiteren Aspekt des Schlagrahmens kann die Mutter eine Halbkugel-Mutter sein, die in einem Halbkugel-Mutterbett am Schlagrahmen gelagert ist.

Gemäß einem weiteren Aspekt kann der Schlagrahmen eine C-Form aufweisen.

### Kurze Beschreibung der Zeichnung

Im Folgenden werden beispielhafte Ausführungsformen der vorliegenden Erfindung unter Bezug auf die Figuren genauer beschrieben, wobei
Fig. 1 eine Front- und eine Seitenansicht eines erfindungsgemäßen modularen chirurgischen Instruments zeigt;
Fig. 2 einen erfindungsgemäßen Werkzeugkopf für ein modulares chirurgisches Instrument und zugehörige Stellglieder zeigt;
Fig. 3 einen Ausschnitt aus Fig. 1 mit einer Verbindungseinrichtung zeigt;
Fig. 4 Beispiele für Werkzeugköpfe zeigt;
Fig. 5 mit Teilfiguren Fig. 5a - Fig. 5j modulare chirurgische Instrumente in verschiedenen Ausführungen zeigt;
Fig. 6 verschiedene Schlagrahmen mit mehreren Rahmenabschnitten stark vereinfacht dargestellt zeigt; und
Fig. 7 Versetzungsebenen illustriert.

Sowohl in der Beschreibung als auch in der Zeichnung gleiche Bezugszeichen für gleiche oder ähnliche Elemente oder Komponenten verwendet. Es ist zudem eine Bezugszeichenliste angegeben, die für alle Figuren gültig ist. Die in den Figuren dargestellten Ausführungen sind lediglich schematisch und stellen nicht notwendigerweise die tatsächlichen Größenverhältnisse dar.

### Ausführliche Beschreibung der Erfindung

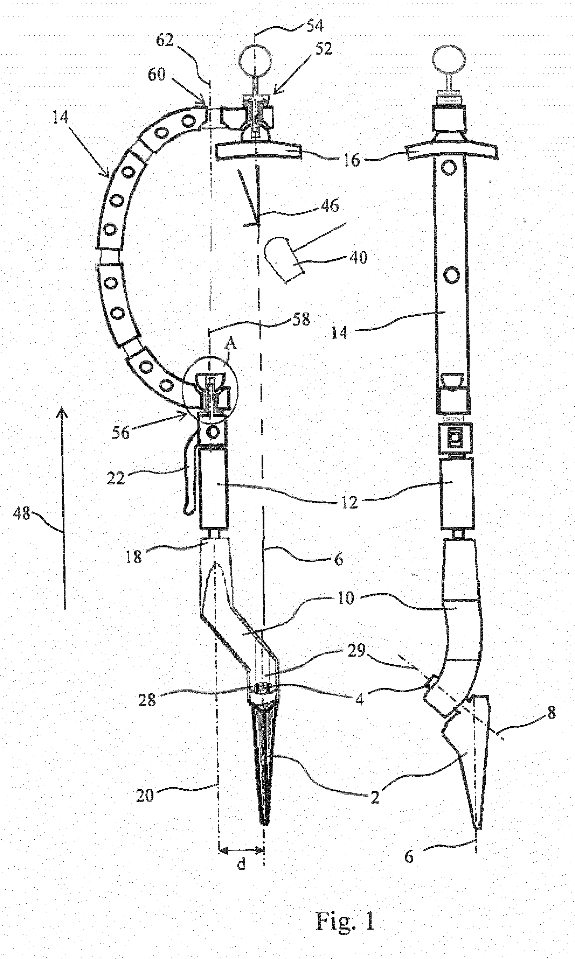

Fig. 1 stellt ein erfindungsgemäßes modulares chirurgisches Instrument (nachfolgend auch einfach als Instrument, als modulares Instrument oder als chirurgisches Instrument bezeichnet) einschließlich optionaler Elemente dar, wobei links in der Figur eine Vorderansicht und rechts eine Seitensicht zu sehen ist und wobei eine vom Instrument arretierte Prothese eingezeichnet ist. Das chirurgische Instrument ist zum Ausschlagen oder Einsetzen, vorzugsweise Ausschlagen, künstlicher Prothesen, insbesondere Gelenkprothesen, z.B. Hüft-, Knie- und/oder Schulterprothesen, gedacht. In der Figur ist eine solche Prothese 2, die einen Prothesenhals 4 aufweist, skizziert. Die Prothese 2 definiert eine Prothesenachse 6, in deren Richtung die Prothese 2 auszuschlagen bzw. einzusetzen ist. Ebenso definiert der Prothesenhals 4 eine Prothesenhalsachse 8, welche nicht parallel zu Prothesenachse 6 verläuft.

Das in Fig. 1 dargestellte modulare chirurgische Instrument umfasst einen Werkzeugkopf 10, der, wie in der Vorderansicht des Instruments auf der linken Seite der Figur zu erkennen ist, einen "Versatz", bevorzugt einen "seitlichen Versatz" wie in der Vorderansicht der Fig. 1, aufweist, damit wird erreicht, dass das Operationsfeld, in der Figur oberhalb der Prothese 2, auf der Prothesenachse 6 nicht durch einen großen Schnitt zugänglich gemacht werden muss und so einen minimal-invasiven Zugang ermöglicht. Genauer wird dieser "Versatz" bzw. "Offset" nachfolgend beschrieben.

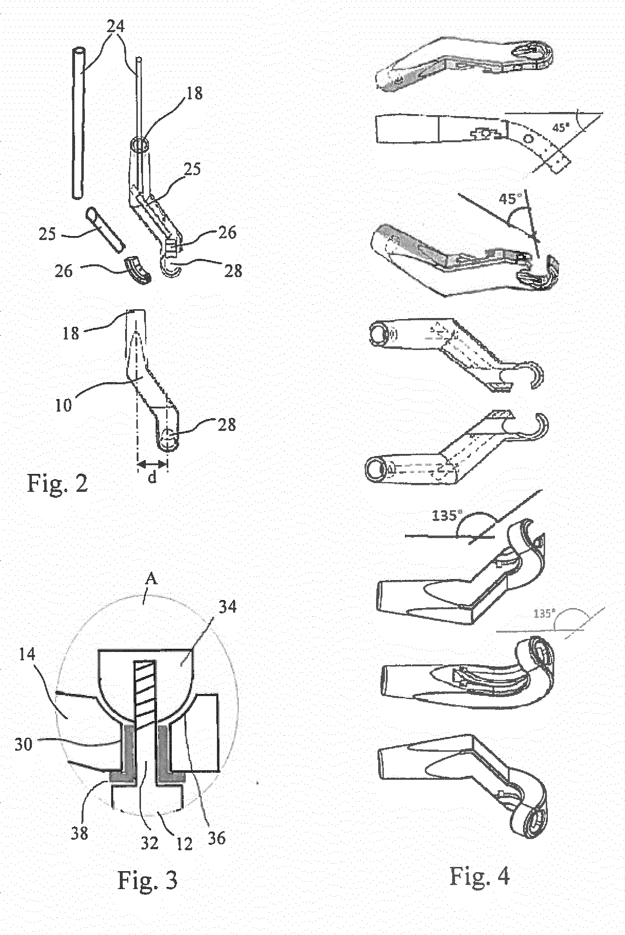

Der Werkzeugkopf 10, vgl. Fig. 2, hat an einem Ende einen Durchgang 28, um den Prothesenhals 4 aufzunehmen, welcher im Durchgang durch eine Verriegelungseinrichtung arretiert wird. Der Durchgang 28 definiert eine Durchgangsachse 29 (in Richtung des Durchgangs). Typischerweise ist der (Umfangs-)Rand des Durchgangs im Wesentlichen ringförmig und die Durchgangsachse entspricht dann der Mittelachse der Ringform, verläuft also senkrecht zu der Ebene in der die Ringform liegt. "Ringförmig" soll hier in einem allgemeinen Sinn, als nicht notwendigerweise kreisringförmig, verstanden werden, z.B. soll eine ovale Form, eine Rechteck oder eine Vieleckform eingeschlossen sein. Der Durchgang 28 ist so eingerichtet, dass bei arretiertem Prothesenhals 4 (wie in der Figur) die Durchgangsachse 29 im Wesentlichen mit er Prothesenhalsachse 8 zusammenfällt; "im Wesentlichen" soll heißen, dass zwischen den beiden Achsen eine geringer Winkel von bis zu 15° bestehen kann, auch kann zwischen den beiden Achsen ein geringer Abstand von bis zu 10% eines Prothesenhalsdurchmessers bestehen.

Der Durchgang kann seitlich offen sein, um das Aufnehmen des Prothesenhalses zu erleichtern bzw. zu ermöglichen. Der Rand des Durchgangs weist dann typischerweise eine Teilringform auf.

Der Werkzeugkopf 10 umfasst weiterhin eine Verriegelungseinrichtung, welche dem Arretieren (Festklemmen) des aufgenommenen Prothesenhalses 4 mittels einer Spannkraft dient. Die Verriegelungseinrichtung kann ein in den Durchgang 28 bewegbar eingreifendes Sperrglied 26 umfassen, das z.B. in seitlichen Nuten geführt wird. Das Sperrglied 26 ist im Wesentlichen senkrecht zur Durchgangsachse 29 bewegbar, so dass ein Querschnitt des Durchgangs 28 geändert werden kann und der Prothesenhals 4 unter Aufbringen der Spannkraft arretiert bzw. festgeklemmt werden kann. Die Spannkraft für die Verriegelungseinrichtung wird an einer Spannaufnahme 18 übertragen und von dort auf die Verriegelungseinrichtung übertragen; z.B. mittels Stellgliedern 24, 25, wie in Fig. 2 skizziert.

Die Spannaufnahme 18 weist eine Achse 20 auf, welche eine Längsrichtung 48 definiert. In Bezug auf die Längsrichtung 48 sind der Durchgang 28 und die Spannaufnahme 18 an entgegengesetzten Enden des Werkzeugkopfes 10 angeordnet. Zwischen der Durchgangsachse 29 und der Längsrichtung 48 besteht bevorzugt ein Winkel von in etwa 45° oder in etwa 135° ("in etwa" heißt, dass Abweichungen von bis zu 5° möglich sind). Durch Parallelverschieben der Durchgangsachse 29 in Längsrichtung 48 wird eine Ebene definiert (in der Vorderansicht auf der linken Seite von Fig. 1 senkrecht zur Zeichenebene). Der seitliche Versatz äußert sich dann darin, dass die Achse 20 der Spannaufnahme 18 nicht in dieser Ebene liegt, dass also zwischen der Achse 20 der Spannaufnahme 18 und der Ebene, die durch die Längsrichtung 48 und die Durchgangsachse 29 definiert wird, ein Abstand d besteht. (In der Vorderansicht ist die Achse 20 der Spannaufnahme 18 beispielhaft nach links von der Ebene, die durch die Längsrichtung 48 und die Durchgangsachse 29 definiert wird, versetzt; natürlich könnte sie ebenso nach rechts versetzt sein.) Dadurch befinden sich die Spannaufnahme 18 und die Bauteile, die die Spannkraft aufbringen, sowohl in Längsrichtung 48 als auch in seitlicher Richtung (quer zur Längsrichtung) außerhalb des Operationsfeldes. Der Abstand d der Achse 20 der Spannaufnahme 18 von der durch die Längsrichtung 48 und die Durchgangsachse 29 definierten Ebene beträgt bevorzugt mindestens 2 cm, weiter bevorzugt mindestens 5 cm. Der Abstand d liegt bevorzugt im Bereich von 2 cm - 20 cm, weiter bevorzugt im Bereich von 5 cm - 15 cm, noch weiter bevorzugt im Bereich von 5 cm - 10 cm.

Die Spannaufnahme ist also zum Durchgang seitlich versetzt, bezogen auf die durch die Durchgangsachse und die Längsrichtung definierte Ebene. Zusätzlich oder ausschließlich kann auch ein Versatz parallel zu dieser Ebene bzw. innerhalb dieser Ebene vorliegen. Beispielsweise kann eine Durchgangs-Längsachse, welche durch den Durchgang 28 und in Längsrichtung 48 verläuft (also verschieden von der Durchgangsachse 29 ist), innerhalb der durch die Durchgangsachse 29 und die Längsrichtung 48 definierten Ebene liegen allerdings einen Abstand zur Achse 20 der Spannaufnahme 18 aufweisen. In der Vorderansicht der Fig. 1 läge dann ein Versatz senkrecht zur Zeichenebene vor; ein Beispiel für einen entsprechenden Werkzeugkopf ist im zweiten Bild von unten der Fig. 4 gezeigt. Ein seitlicher Versatz impliziert klarerweise einen solchen Abstand. Auch dieser Abstand zwischen Durchgangs-Längsachse und Achse 20 der Spannaufnahme beträgt bevorzugt mindestens 2 cm, weiter bevorzugt mindestens 5 cm, und liegt bevorzugt im Bereich von 2 cm - 20 cm, weiter bevorzugt im Bereich von 5 cm - 15 cm, noch weiter bevorzugt im Bereich von 5 cm - 10 cm.

In Fig. 1 ist weiterhin eine optionale Spannbaugruppe (bzw. ein Spannrahmen) 12, d.h. eine Baugruppe, die geeignet ist die Spannkraft zu erzeugen, dargestellt, die an der Spannaufnahme lösbar mit dem Werkzeugkopf 10 verbunden ist; "optional", da die Spannbaugruppe im Prinzip auch mit Werkzeugkopf und/oder dem (weiter unten besprochenen) Schlagrahmen 14 integriert sein kann, bevorzugt wird jedoch ein modularer Aufbau. Die Spannkraft wird mittels eines Spannelements beaufschlagt und mittels Stellgliedern an den Werkzeugkopf 10 übertragen. Beispielsweise kann die Spannkraft mittels eines zu betätigenden Spannhebels 22 mit exzentrisch ausgebildetem Hebelkopf (nicht dargestellt), der auf ein Stellglied wirkt, erzeugt werden.

Die Spannbaugruppe 12 weist eine Achse auf, die, wenn die Spannbaugruppe 12 mit Werkzeugkopf 10 an der Spannaufnahme 18 verbunden ist, mit der Achse 20 der Spannaufnahme 18 zusammenfällt, so dass sich der Versatz auf die Spannbaugruppe 12 überträgt und auch die typischerweise als längliches Bauteil ausgeführte Spannbaugruppe 12 nicht in das Operationsfeld ragt. Weiterhin kann die Spannbaugruppe 12 eingerichtet sein, eine veränderliche Voreinstell-Verschiebung der Verriegelungseinrichtung zu erreichen (d.h. ein einstellbares Verschieben der Verriegelungseinrichtung vor Aufbringen der eigentlichen Spannkraft, welche den Prothesenhals festklemmt), um für verschiedene Prothesenhälse mit unterschiedlichen Abmessungen bzw. Durchmessern verwendet werden zu können. Im obigen Beispiel kann hierfür kann ein Stellgewinde in der Spannbaugruppe vorgesehen sein, dass den Abstand des Spannhebels 22 von der Spannaufnahme 18 ändert und so eine Vorspannung auf das Stellglied bewirkt.

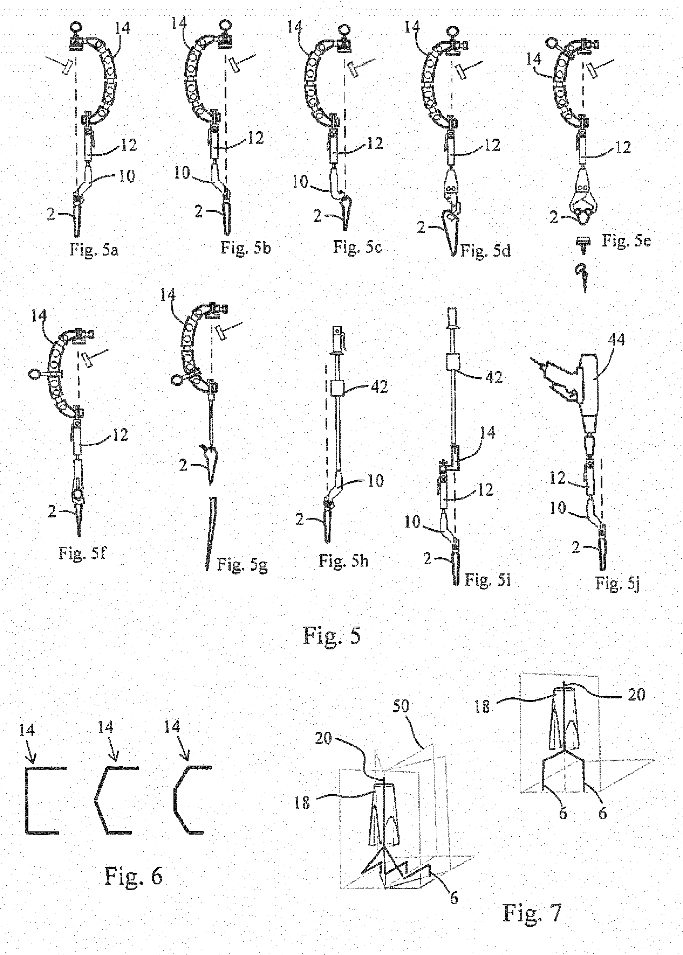

In Fig. 1 ist weiterhin ein, hier beispielsweise C-förmiger, Schlagrahmen 14 dargestellt, der mit der Spannbaugruppe 12 lösbar verbunden ist und eine erste Verbindungseinrichtung 52 mit einer ersten Achse 54 für eine Schlagvorrichtung zum Übertragen einer Schlagkraft aufweist, die mit Hilfe der Schlagvorrichtung erzeugt wird und die entlang der ersten Achse 54 wirken soll, wobei die Schlagvorrichtung mittels der ersten Verbindungseinrichtung mit dem Schlagrahmen verbunden ist. Der Schlagrahmen 14 ist optional, da die Schlagvorrichtung auch direkt an der Spannbaugruppe 12 angebracht oder in diese integriert sein könnte (siehe z.B. Fig. 5h). Der erfindungsgemäße Schlagrahmen kann zusammen mit einem Werkzeugkopf mit Versatz verwendet werden (Fig. 5a - Fig. 5c) oder unabhängig davon, z.B. kann der Schlagrahmen auch mit herkömmlichen Werkzeugköpfen unterschiedlicher Art (Fig. 5d - Fig. 5g) gekoppelt sein.

Die Schlagvorrichtung kann z.B. eine Schlagplatte 16, welche insbesondere plan bzw. eben sein kann, bevorzugt jedoch konkav ist (wie in Fig. 1 dargestellt), einen Gleithammer 42 (siehe Fig. 5h und Fig. 5i) oder eine Pneumatik-Schlagmaschine umfassen. Auf eine Schlagplatte 16 wird z.B. mit einem Hammer 40 geschlagen um die Schlagkraft zu erzeugen. Eine konkave, d.h. konkav gekrümmte (z.B. entsprechend eines Ausschnitts einer Kugeloberfläche vom Inneren der Kugel aus gesehen), Schlagplatte 16 ist vorteilhaft, da ein Hammerschlag so zwangsläufig zentriert wird.

Die lösbare Verbindung der Spannbaugruppe 12 mit dem Schlagrahmen 14 erfolgt mittels einer zweiten Verbindungseinrichtung 56 mit einer zweiten Achse 58, wobei, im verbundenen Zustand, die zweite Achse 58 mit der Achse der Spannbaugruppe 12 und entsprechend der Achse 20 der Spannaufnahme 18 zusammenfällt. Bevorzugt sind die erste Achse 54 und die zweite Achse 58 parallel und voneinander beabstandet, weiter bevorzugt soll die erste Verbindungseinrichtung 52 vom Durchgang 28 aus gesehen in etwa in Längsrichtung 48 angeordnet sein, so dass die erste Achse 54 bei arretiertem Prothesenhals 4 mit der Prothesenachse 6 der am Prothesenhals 4 arretierten Prothese 2 im Wesentlichen zusammenfällt.

Der Abstand der ersten von der zweiten Achse entspricht bevorzugt dem Abstand d der Achse 20 der Spannaufnahme 18 von der durch die Längsrichtung 48 und die Durchgangsachse 8 definierten Ebene, beträgt also bevorzugt mindestens 2 cm, weiter bevorzugt mindestens 5 cm. Analog liegt der Abstand der beiden Achsen bevorzugt im Bereich von 2 cm - 20 cm, weiter bevorzugt 5 cm - 15 cm, noch weiter bevorzugt im Bereich von 5 cm - 10 cm. Auf diese Weise fällt die erste Achse 54, entlang welcher die Schlagkraft wirkt, mit der Prothesenachse 6, entlang welcher die Prothese 2 ausgeschlagen bzw. eingesetzt werden soll, zusammen; quer wirkende Kräfte, die durch den seitlichen Versatz des Werkzeugkopfes 10 entstehen würden und die z.B. zu Beschädigungen am Knochen führen könnten, treten also nicht oder nur in geringem Umfang auf und können durch eine entsprechende Gestaltung der Verbindungseinrichtungen weiter verringert werden.

Eine erste Möglichkeit, siehe Fig. 5i, dies zu realisieren umfasst einen L-förmigen Schlagrahmen. Ein erster Schenkel der L-Form, an dem die erste Verbindungseinrichtung angeordnet ist, verläuft dabei entlang der ersten Achse. An einem zweiten Schenkel der L-Form ist die Spannbaugruppe 12 lösbar verbunden. Die Schlagvorrichtung 16, beispielsweise ein Gleithammer 42, kann in Verlängerung des ersten Schenkels der L-Form an der ersten Verbindungseinrichtung angebracht werden.

Eine bevorzugte zweite Ausführungsform des Schlagrahmens umfasst einen Schlagrahmen 14 mit einem ersten und einem zweiten Schenkel, wobei an einem Ende des ersten Schenkels die erste Verbindungseinrichtung 52 mit der ersten Achse 54 angeordnet ist und an einem Ende des zweiten Schenkels die zweite Verbindungseinrichtung 56 mit der zweiten Achse 58 angeordnet ist. (Anmerkung: Die Schenkel dieser zweiten Möglichkeit werden lediglich als "Schenkel" bezeichnet, im Unterschied zu den "Schenkeln der L-Form" der vorstehenden ersten Möglichkeit.) Die erste Achse 54 und die zweite Achse 58 verlaufen parallel und sind voneinander beabstandet; die erste Verbindungseinrichtung 52 ist zum Befestigen der Schlagvorrichtung 16 vorgesehen und die zweite Verbindungvorrichtung 56 ist zum Befestigen der Spannbaugruppe 12 vorgesehen. Ein Raum zwischen den Enden der Schenkel bleibt in Richtung der beiden Achsen 54, 58 frei, so dass dieser Raum dem Operateur zur Verfügung steht; etwa um mit einem Hammer 40 auf eine Schlagplatte 16 zu schlagen, wie in den Figuren dargestellt.

Bei der zweiten Schlagrahmen-Ausführungsform ist bevorzugt eine dritte Verbindungseinrichtung 60 mit einer dritten Achse 62 am ersten Schenkel angeordnet, und zwar so, dass die dritte Achse 62 mit der zweiten Achse 58 zusammenfällt. Dadurch kann ein entsprechender Schlagrahmen auch zusammen mit Werkzeugköpfen ohne seitlichen Versatz zu Einsatz kommen; vgl. Fig. 5d, Fig. 5e und Fig. 5f. Erfindungsgemäß kann der Schlagrahmen gemäß der zweiten Schlagrahmen-Ausführungsform kann also unabhängig vom Werkzeugkopf mit Versatz Verwendung finden. Die dritte Verbindungseinrichtung kann entsprechend der ersten und der zweiten Verbindungseinrichtung gestaltet sein; siehe unten.

Konkret lässt sich der Schlagrahmen 14 gemäß der zweiten Schlagrahmen-Ausführungsform beispielsweise realisieren indem der Schlagrahmen 14 ein C-förmig oder U-förmig gebogenes Rahmenelement umfasst, dessen Endabschnitte den ersten und den zweiten Schenkel bilden, wobei ein Schenkel länger als der andere Schenkel ist, die erste Verbindungseinrichtung am Ende des längeren Schenkels angeordnet ist und die zweite Verbindungseinrichtung am Ende des kürzeren Schenkels angeordnet ist. Bevorzugt, da flexibler und vielseitiger einsetzbar, ist jedoch, dass der Schlagrahmen 14 mehrere Rahmenabschnitte umfasst, wobei der erste und der zweite Schenkel zwei Rahmenabschnitte bilden, die entweder direkt oder, bevorzugt, durch weitere (mindestens einen) Rahmenabschnitte miteinander verbunden sind, die sich zumindest teilweise in die Richtung erstrecken, die durch die erste bzw. zweite Achse definiert ist. Die Enden des ersten und des zweiten Schenkels verlaufen bevorzugt im Wesentlichen senkrecht zu der ersten und der zweiten Achse. Die einzelnen Rahmenabschnitte können geradlinig oder gekrümmt (wie in Fig. 1) sein und z.B. als längliche Metallelemente ausgebildet sein. In Fig. 6 sind, stark vereinfacht, Schlagrahmen 14 mit 3, 4 bzw. 5 Rahmenabschnitten dargestellt. Sind mehrere geradlinige Rahmenabschnitte entsprechend angeordnet oder werden gekrümmte Rahmenabschnitte verwendet, ergibt sich im Wesentlichen eine besonders bevorzugte C-Form des Schlagrahmens.

Bevorzugt sind die Rahmenabschnitte fest bzw. starr miteinander verbunden, um eine hinreichende Steifheit des Schlagrahmens zu gewährleisten. Alternativ können die Rahmenabschnitte verstellbar und fixierbar miteinander verbunden sein, z.B. mittels Schraubverbindungen, dadurch kann der Schlagrahmen 14 in seiner Form geändert werden und an unterschiedliche Werkzeugköpfe und Spannbaugruppen angepasst werden. Weiterhin kann der Schlagrahmen mit Anbringungsmöglichkeiten, z.B. Aussparrungen oder Löchern, für einen Handgriff zum Halten des Schlagrahmens bzw. des chirurgischen Instruments versehen sein (siehe z.B. Fig. 5e, Fig. 5f und Fig. 5g).

Für die Verbindungseinrichtungen (erste, zweite und dritte) gibt es jeweils verschiedenste Realisierungsmöglichkeiten, z.B. Schraubverbindungen, Klemmverbindungen, Nut-Feder-Systeme, Bajonettverschlüsse usw. Eine bevorzugte besteht darin, dass zumindest eine (bevorzugt beide) Verbindungseinrichtung ein Durchgangsloch 30 im Schlagrahmen 14 umfasst, welches sich in Richtung der ersten bzw. zweiten Achse erstreckt; die Spannbaugruppe bzw. die Schlagvorrichtung wird dann befestigt indem z.B. eine Schraube oder ein Schraubbolzen durch das jeweilige Durchgangsloch geführt und verschraubt wird.

Dies ist beispielsweise in dem in Fig. 3 dargestellten Detail-Ausschnitt A aus Fig. 1 gezeigt. Durch das Durchgangsloch 30 im Schlagrahmen 14 ist eine Verbindungsschraube 32 geführt, die mit der Spannbaugruppe verbunden bzw. in diese integriert ist. Die Verbindungsschraube 32 wird am Schlagrahmen 14 mittels einer Mutter 34, in der Figur beispielsweise einer Halbkugel-Mutter, die in einem Halbkugel-Mutterbett 36 gelagert ist, festgeschraubt. Weiter ist in dem Durchgangsloch 30 eine Elastomerhülse (Elastomerring) 38 angeordnet, welche die Verbindungsschraube 32 umgibt, um so quer wirkende Kräfte zu dämpfen, da z.B. der Schlag mit einem Hammer normalerweise etwas schräg erfolgt und dann auch eine Kraft quer zur Längsrichtung bewirkt; dies ist in Fig. 1 durch ein Diagramm 46 der Kraftvektoren angedeutet. Bevorzugt besteht die Elastomerhülse 38 aus Silikon oder umfasst Silikon. Die Verbindungseinrichtung ist hier also durch eine Baugruppe gebildet, man hat sozusagen einen Verbindungseirichtungs-Komplex.

Die Verbindungseinrichtung der Ausführungsform gemäß Fig. 3 ist nicht-fest in dem Sinne, dass die damit hergestellte Verbindung in einige Richtungen elastisch ist (z.B. quer zur Achse der Verbindungseinrichtung); sie ist sozusagen bedingt-fest - in Abhängigkeit von der Richtung der Krafteinwirkung. Hier sind auch Verbindungseinrichtungen bzw. Verbindungen möglich, die fest sind, also in keine Richtung elastisch sind (oder dämpfend wirken). Beispielsweise eine Schraubverbindung ohne Elastomerhülse oder sonstiges elastisches Element. Ebenso sind nicht lösbare Verbindungen denkbar; z.B. können die Spannbaugruppe und/oder die Schlagvorrichtung an den Schlagrahmen gelötet oder geschweißt sein. Bevorzugt sind jedoch lösbare Verbindungseinrichtungen bzw. Verbindungen.

In Fig. 4 sind unterschiedliche Beispiele für Werkzeugköpfe dargestellt, die alle mit Ausnahme des zweiten von unten, der einen Versatz nach oben aufweist, einen seitlichen Versatz aufweisen. Der jeweilige Durchgang ist bei einigen der Beispiele seitlich offen. Weiterhin sind unterschiedliche Winkel (45°, 135°) zwischen Durchgangsachse und Achse der Spannaufnahme angedeutet. Ebenso sind Beispiele (1. und 3. Bild bzw. 4. und 5. Bild) gezeigt, die sich lediglich dadurch unterscheiden, dass der Versatz in Bezug auf eine Vorderansicht in verschiedene Richtungen erfolgt (links, rechts, oben: diese Richtungen beziehen sich jeweils auf die Darstellungen in der Figur). Es ist möglich, alle diese unterschiedlichen Werkzeugköpfe zusammen mit dem Schlagrahmen der vorliegenden Erfindung zu verwenden.

Fig. 5, die aus Teilfiguren Fig. 5a - Fig. 5j besteht, stellt chirurgische Instrumente in verschiedenen Ausführungen dar. Die Figur soll insbesondere den modularen Charakter des chirurgischen Instruments der vorliegenden Erfindung illustrieren und die daraus folgenden Einsatzmöglichkeiten bei verschiedenen Prothesen und bei unterschiedlichen Zugangsarten. Fig. 5a, Fig. 5b und Fig. 5c zeigen die Verwendung unterschiedlicher erfindungsgemäßer Werkzeugköpfe 10, die einen seitlichen Versatz aufweisen, wobei jeweils der Schlagrahmen 14 und die Spannbaugruppe 12 zum Einsatz kommen. Fig. 5d, Fig. 5e, Fig. 5f und Fig. 5g zeigen die Verwendung eines Schlagrahmens 14, der die dritte Verbindungseinrichtung (hier ein Durchgangsloch im Schlagrahmen) aufweist, wobei jeweils ein (im Stand der Technik bekannter) Werkzeugkopf ohne seitlichen Versatz zum Einsatz kommt. In Fig. 5d und Fig. 5e wird die jeweilige Prothese 2 mit einer Spreizzange (Fig. 5d) oder Klemmzange (Fig. 5e) gegriffen. In Fig. 5f kommt ein geradliniger Werkzeugkopf mit seitlich offenem Durchgang zum Einsatz. In Fig. 5g ist die Prothese 2 über eine Schraubverbindung mit einer Stange verbunden, die mit dem Schlagrahmen 14 an der zweiten Verbindungseinrichtung verbunden ist. Die Schlagkraft wird in diesen Fällen (Fig. 5d, Fig. 5e, Fig. 5f und Fig. 5g) mittels einer Schlagplatte 16 (und einem Hammer) erzeugt, die an der dritten Verbindungseinrichtung verbunden ist. In Fig. 5e und Fig. 5g sind weitere Prothesenarten unterhalb der mit dem chirurgischen Instrument verbundenen Prothese 2 angedeutet. Fig. 5h zeigt die Verwendung eines Werkzeugkopfes 10 mit seitlichem Versatz, wobei die Schlagkraft durch einen Gleithammer 42 mit integrierter Spannbaugruppe erzeugt wird, der direkt mit dem Werkzeugkopf 10 verbunden ist. Fig. 5i zeigt die Verwendung eines Werkzeugkopfes 10 mit seitlichem Versatz und eines L-förmigen Schlagrahmens 14, wobei die Schlagkraft mit einem Gleithammer 42, der an einem Schenkel der L-Form verbunden ist, erzeugt wird und eine Spannbaugruppe 12 am anderen Schenkel der L-Form verbunden ist. Fig. 5j zeigt die Verwendung eines Werkzeugkopfes 10 mit seitlichem Versatz, wobei die Schlagkraft mit einer Pneumatik-Schlagmaschine 44 erzeugt wird, der über eine Spannbaugruppe 12 mit dem Werkzeugkopf 10 verbunden ist. Bei den Anordnungen gemäß Fig. 5h und Fig. 5j fällt die Achse der Schlagkrafteinwirkung nicht mit der Prothesenachse (die der Ausschlagachse entspricht) zusammen, beim Ausschlagen können also Querkräfte auftreten.

Fig. 7 stellt die "Versetzungsebenen" stark vereinfacht dar. Hierbei ist der Werkzeugkopf teilweise dargestellt, wobei insbesondere die Spannaufnahme 18 und deren Achse 20 dargestellt sind. Ebenso ist die von der Achse 20 der Spannaufnahme 18 beabstandete Prothesenachse 6 dargestellt, wobei letztere bei seitlichem Versatz außerhalb der durch die Durchgangsachse und die Längsrichtung definierten Ebene 50 liegt bzw. in einem Fall im Teilbild links unten innerhalb dieser Ebene (entsprechend den im zweiten Bild von unten in Fig. 4 dargestellten Werkzeugkopf). Die skizzierten Achsen im Teilbild rechts oben entsprechen Werkzeugköpfen, bei denen der Winkel zwischen Durchgangsachse und Längsrichtung in etwa 45° beträgt, beim Teilbild links unten beträgt dieser Winkel in etwa 135°.

Zusammengefasst sind der Werkzeugkopf 10 und der Schlagrahmen 14 bevorzugt so eingerichtet, dass einerseits die erste Achse 54 mit der Prothesenachse 6 zusammenfällt und andererseits die zweite Achse 58 mit der Achse 20 der Spannaufnahme 18 und, gegebenenfalls, der dritten Achse 62 zusammenfällt, wobei die erste und die zweite Achse voneinander beabstandet sind; so wird erreicht, dass ein minimal-invasiver Eingriff möglich ist und dass die Krafteinwirkung entlang der Prothesenachse erfolgt.

### Bezugszeichenliste

- 2: Prothese
- 4: Prothesenhals
- 6: Prothesenachse
- 8: Prothesenhalsachse
- 10: Werkzeugkopf
- 12: Spannbaugruppe
- 14: Schlagrahmen
- 16: (konkave) Schlagplatte
- 18: Spannaufnahme
- 20: Achse der Spannaufnahme
- 22: Spannhebel / Spannelement
- 24: Stellglied
- 25: Stellglied
- 26: Sperrglied
- 28: Durchgang
- 29: Durchgangsachse
- 30: Durchgangsloch
- 32: Verbindungschraube
- 34: Mutter / Halbkugel-Mutter
- 36: Halbkugel-Mutterbett
- 38: Elastomerhülse
- 40: Hammer
- 42: Gleithammer
- 44: Pneumatik-Schlagmaschine
- 46: Diagramm der Kraftvektoren
- 48: Längsrichtung
- 50: durch Durchgangsachse und Längsrichtung definierte Ebene
- 52: erste Verbindungseinrichtung
- 54: erste Achse
- 56: zweite Verbindungseinrichtung
- 58: zweite Achse
- 60: dritte Verbindungseinrichtung
- 62: dritte Achse
- A: Ausschnitt
- d: Abstand

## Patentansprüche

1. Modulares chirurgisches Instrument zum Ausschlagen oder Einsetzen einer Prothese (2), die eine Prothesenachse (6) definiert und die einen Prothesenhals (4) aufweist, der eine Prothesenhalsachse (8) definiert, die nicht parallel zur Prothesenachse verläuft, umfassend einen Werkzeugkopf (10) mit einem Durchgang (28) zur Aufnahme des Prothesenhalses (4), einer Verriegelungseinrichtung zur Arretierung des aufgenommenen Prothesenhalses (4) und einer Spannaufnahme (18), die eine Achse (20) aufweist, zum Übertragen einer Spannkraft für die Verriegelungseinrichtung,
wobei der Durchgang (28) und die Spannaufnahme (18) in Bezug auf eine durch die Achse (20) der Spannaufnahme definierten Längsrichtung (48) an entgegengesetzten Enden des Werkzeugkopfes (10) angeordnet sind, und
wobei der Durchgang (28) eine Durchgangsachse (29) definiert, die bei arretiertem Prothesenhals (4) im Wesentlichen mit der Prothesenhalsachse (8) zusammenfällt;
wobei die Achse (20) der Spannaufnahme (18) von der durch die Längsrichtung und die Durchgangsachse (29) definierten Ebene (50) beabstandet ist und/oder die Achse (20) der Spannaufnahme (18) von einer Durchgangs-Längsachse, die in Längsrichtung (48) und durch den Durchgang (28) verläuft, beabstandet ist.

2. Modulares chirurgisches Instrument gemäß Anspruch 1, wobei der Abstand (d) der Achse (20) der Spannaufnahme (18) von der durch die Längsrichtung (48) und die Durchgangsachse (29) definierten Ebene im Bereich von 2 cm - 20 cm, bevorzugt 5 cm - 15 cm, liegt.

3. Modulares chirurgisches Instrument gemäß einem der vorstehenden Ansprüche, wobei die Verriegelungseinrichtung ein in den Durchgang (28) bewegbar eingreifendes Sperrglied (26) zur Arretierung des Prothesenhalses (4) umfasst, wobei die Durchgangsachse (29) im Wesentlichen senkrecht zu einer Bewegungsrichtung des Sperrglieds verläuft.

4. Modulares chirurgisches Instrument gemäß einem der vorstehenden Ansprüche, weiterhin umfassend eine Spannbaugruppe (12), die mit dem Werkzeugkopf (10) an der Spannaufnahme (18) lösbar verbunden ist und die ein Spannelement (22) zur Beaufschlagung einer Spannkraft und Stellglieder zur Übertragung der Spannkraft aufweist.

5. Modulares chirurgisches Instrument gemäß Anspruch 4, weiterhin umfassend einen Schlagrahmen (14), der mit der Spannbaugruppe (12) lösbar verbunden ist und der eine erste Verbindungseinrichtung (52) mit einer ersten Achse (54) für eine Schlagvorrichtung zum Übertragen einer Schlagkraft aufweist.

6. Modulares chirurgisches Instrument gemäß Anspruch 5, umfassend die Schlagvorrichtung, die an der ersten Verbindungseinrichtung (52) angebracht ist, wobei die Schlagvorrichtung eine Schlagplatte (16), einen Gleithammer (42) oder eine Pneumatik-Schlagmaschine (44) umfasst und so eingerichtet ist, dass die Schlagkraft entlang der ersten Achse (54) wirkt.

7. Modulares chirurgisches Instrument gemäß einem der Ansprüche 5 - 6, wobei die erste Verbindungseinrichtung (52) oder eine feste Verbindung vom Durchgang (28) aus gesehen in Längsrichtung (48) angeordnet ist, so dass die erste Achse (54) bei arretiertem Prothesenhals (4) mit der Prothesenachse (6) zusammenfällt.

8. Modulares chirurgisches Instrument gemäß einem der Ansprüche 5 - 7, wobei der Schlagrahmen (14) einen ersten und einen zweiten Schenkel umfasst;
wobei an einem Ende des ersten Schenkels die erste Verbindungseinrichtung (52) oder eine feste Verbindung, bevorzugt die Verbindungseinrichtung, mit der ersten Achse (56) für die Schlagvorrichtung angeordnet ist;
wobei an einem Ende des zweiten Schenkels eine zweite Verbindungseinrichtung (56) oder eine feste Verbindung, bevorzugt die Verbindungseinrichtung, mit einer zur ersten Achse (54) parallelen zweiten Achse (58) für die Spannbaugruppe (12) angeordnet ist;
wobei ein Raum zwischen den Enden der Schenkel in Richtung der ersten (54) und der zweiten Achse (58) freibleibt; und
wobei die erste Achse (54) und die zweite Achse (58) voneinander beabstandet sind.

9. Modulares chirurgisches Instrument gemäß einem der Ansprüche 5 - 7, wobei der Schlagrahmen (14) eine L-Form aufweist, wobei die Spannbaugruppe (12) an einem zweiten Schenkel der L-Form mittels einer zweiten Verbindungeinrichtung oder einer festen Verbindung lösbar verbunden ist und wobei die erste Verbindungseinrichtung oder eine feste Verbindung für die Schlagvorrichtung an einem ersten Schenkel der L-Form angeordnet ist.

10. Schlagrahmen für ein modulares chirurgisches Instrument zum Ausschlagen oder Einsetzen einer Prothese (2), die eine Prothesenachse (6) definiert und die einen Prothesenhals (4) aufweist, der eine Prothesenhalsachse (8) definiert, die nicht parallel zur Prothesenachse verläuft, aufweisend einen ersten und einen zweiten Schenkel;
wobei an einem Ende des ersten Schenkels eine erste Verbindungseinrichtung (52) mit einer ersten Achse (54) für eine Schlagvorrichtung angeordnet ist;
wobei an einem Ende des zweiten Schenkels eine zweite Verbindungseinrichtung (56) mit einer zur ersten Achse (54) parallelen zweiten Achse (58) für eine Spannbaugruppe (12) angeordnet ist;
wobei ein Raum zwischen den Enden der Schenkel in Richtung der ersten und zweiten Achse (54, 58) freibleibt; und
wobei die erste Achse (54) und die zweite Achse (58) voneinander beabstandet sind.

11. Schlagrahmen gemäß Anspruch 10, wobei der Abstand (d) zwischen der ersten Achse (54) und der zweiten Achse (58) im Bereich von 2 cm - 20 cm, bevorzugt 5 cm - 10 cm, liegt.

12. Schlagrahmen gemäß einem der Ansprüche 10 - 11, wobei der Schlagrahmen (14) mehrere Rahmenabschnitte umfasst,
wobei der erste Schenkel und der zweite Schenkel Rahmenabschnitte bilden, deren Enden sich im Wesentlichen senkrecht zu der ersten und der zweiten Achse (54, 58) erstrecken, und wobei sich mindestens ein weiterer Rahmenabschnitt, der den ersten Schenkel und den zweiten Schenkel verbindet, zumindest teilweise in die durch die erste und die zweite Achse definierte Richtung erstreckt.

13. Schlagrahmen gemäß einem der Ansprüche 10 - 12, wobei am ersten Schenkel zusätzlich eine dritte Verbindungseinrichtung (60), die bevorzugt ein Durchgangsloch am Schlagrahmen umfasst, mit einer dritten Achse (62) für eine Schlagvorrichtung angeordnet ist, wobei die dritte Achse (62) mit der zweiten Achse (58) zusammenfällt.

14. Schlagrahmen gemäß einem der Ansprüche 10 - 13, wobei an der ersten Verbindungseinrichtung (52) eine Schlagplatte (16), insbesondere eine ebene oder ein konkave Schlagplatte, oder ein Gleithammer (42) angebracht ist.

15. Schlagrahmen gemäß einem der Ansprüche 10 - 14, wobei die erste und/oder die zweite Verbindungseinrichtung jeweils ein Durchgangsloch (30) im Schlagrahmen (14), eine Elastomerhülse (38), bevorzugt aus Silikon, die in dem Durchgangsloch (30) angeordnet ist und eine Verbindungsschraube (32), die sich durch das Verbindungsloch erstreckt und die mittels einer Mutter befestigt wird, wobei die Mutter eine Halbkugel-Mutter (34) ist, die in einem Halbkugel-Mutterbett (36) am Schlagrahmen (14) gelagert ist, umfasst.
